# EUROPEAN PATENT SPECIFICATION

(11) **EP 4 408 208 B1**
(45) Date of publication and mention of the grant of the patent: **03.09.2025**
(21) Application number: 22736291.0
(22) Date of filing: 01.07.2022
(51) Int. Cl.: A24F 40/40, A24F 40/485

(54) **AEROSOL GENERATING DEVICE**
AEROSOLERZEUGUNGSVORRICHTUNG
DISPOSITIF DE GÉNÉRATION D'AÉROSOL

(30) Priority: 29.09.2021 EP 21199721
(43) Date of publication of application: 07.08.2024
(73) Proprietor: JT International SA, 1202 Geneva (CH)
(72) Inventor: BOUCHUIGUIR, Layth Sliman, 1293 Bellevue (CH)
(74) Representative: Gill Jennings & Every LLP
(86) International application number: PCT/EP2022/068345
(87) International publication number: WO 2023/051964

(56) References cited:
- WO-A1-2021/037822
- WO-A2-2013/102609
- US-A1- 2020 345 066

## Description

### FIELD

The present invention relates to a sealing element for use in an aerosol generating device, the sealing element having a valve to prevent the flow of aerosol into the aerosol generating device.

### BACKGROUND

Electronic cigarettes, otherwise known as vapes, are becoming a popular alternative to smoking traditional tobacco cigarettes. Tobacco vapour (T-vapour) devices are a type of aerosol-generating device which heat tobacco at a lower temperature compared to traditional cigarettes. These devices are often referred to as heat not burn devices.

In T-vapour devices the vaporisable material is usually provided in the form of sticks of aerosol-generating material, usually a tobacco-containing material. These sticks are designed to be heated and not combusted as standard cigarettes are. A part of the stick comprising the aerosol-generating material can be inserted into a chamber of the aerosol-generating device which includes a heater for heating the inserted part of the stick. When heated, the aerosol generating material forms a vapour, otherwise referred to as an aerosol, which then passes through the stick and is inhaled by the user. After consumption the stick can be removed and disposed of until a next consumption of a fresh stick by a user.

Not all of the vaporised material will have chance to exit during the puff. For instance, between and after puffs the heater may still be functional for a short period of time thereby creating further aerosol that is not inhaled by the user. Problems can occur through this aerosol from the heater escaping from the heating area and entering other parts of the device. This leakage and ingress of aerosol from the heating region of the device can occur between puffs and shortly after the device has been used. Over time this can cause a build-up of residue that can cause damage to the electronics and mechanical components of the device thereby leading to malfunctions of these components. The present invention is aimed at avoiding these issues.

WO2021/037822 discloses an aerosol generating device with sealing element.

### SUMMARY OF INVENTION

According to a first aspect there is provided an aerosol generation device, comprising: a chamber for receiving a consumable cartridge, the consumable cartridge comprising an aerosol generating material, the chamber comprising: a first end through which the consumable cartridge is received; a second end; and an internal side wall extending from the first end to the second end, the side wall defining the chamber between the first and second ends; a sealing element located in the chamber, the sealing element configured to provide a fluidic seal between the consumable cartridge, when received in the chamber, and the internal side wall of the chamber, the sealing element comprising: a valve, the valve configured such that, flow of fluid is prevented through the valve in a direction towards the first end of the chamber.

Advantageously, the sealing element within the chamber tightly surrounds the consumable cartridge thereby providing a fluidic seal between the cartridge and the chamber. Fluid flow in the chamber may be permitted through the valve in a direction towards the second end of the chamber but prevented through the valve in a direction towards the first end of the chamber. The valve prevents the flow of fluid along the chamber (i.e. in the region surrounding the consumable cartridge) in a direction from the second end to the first end of the chamber, whereas it may allow the flow of fluid along the chamber from the first end to the second end of the chamber.

In this way, when a user takes a puff on the cartridge, air enters the device through the chamber via its first end (i.e. in the gap between the cartridge and the wall of the chamber) and passes through the valve and into a heating area before entering the consumable cartridge forcing the aerosol, generated from the aerosol generating substance, up through the cartridge and into the user's mouth. However, the remaining aerosol in the heating area is prevented from passing back up through the chamber after the puff has finished due to the seal provided by the sealing element and the one-way valve preventing flow through the chamber in that direction. As the valve acts as a one way valve it only allows the flow of fluid through the chamber towards its second end.

Advantageously, this can prevent ingress of aerosol, otherwise referred to as vapour, into the internal components of the device thereby reducing damage caused by the aerosol, particularly when the device is in a standby state between puffs and after use.

The chamber may be referred to as a cavity, i.e. it is a space in which at least part of the consumable cartridge is received. The first end of the chamber may be referred to the mouthpiece end of the chamber i.e. it is proximal to where the mouthpiece or end of the consumable cartridge which the user places in their mouth when using the device, whereas the second end of the chamber may be distal to the mouthpiece. The first end of the chamber may provide an opening in which the consumable cartridge is received.

Preferably, the sealing element is comprised of a plastic material. For instance, the sealing element may be comprised of Polyether ether ketone (PEEK). Advantageously, this enables the sealing element to withstand chemical and thermal stresses, such as high temperatures. Alternately, or in addition, the sealing element may be comprised of silicone. In other arrangements, the sealing element may be formed of any type of plastic material, ceramic, or rubber.

In some arrangements, a first axis is defined between the first and second ends of the chamber, and wherein the chamber has a width perpendicular to the first axis, wherein the sealing element has a cross section in a plane perpendicular to the first axis, the cross section having an outline that is substantially matched to the width of the chamber thereby to provide a seal between the sealing element and the internal side wall of the chamber.

In this way, the sealing element is fitted into the chamber through having a shape and size that is substantially matched to that of the chamber. This helps provide a tight fluidic seal preventing leakage of aerosol.

Preferably, the sealing element has an annular cross section in a plane perpendicular to the first axis, the annular cross section comprising an inner diameter that is configured to receive the consumable cartridge, and comprising an outer diameter that is substantially matched to the width of the chamber thereby to provide a seal between the sealing element and the internal side wall of the chamber. Advantageously, the sealing element is both matched to the size and shape of the consumable cartridge and the size and shape of the chamber. This provides a secure fluidic seal preventing leakage.

The inner diameter may be matched to the size of the consumable cartridge. The void formed by the inner diameter of the sealing element may provide a region in which the consumable cartridge is received. In other words, the sealing element may be ringed shape with the consumable cartridge housed within the centre of the ring.

In other arrangements, the sealing element may have a cross section that is a different shape to an annulus. Consumable cartridges are typically cylindrical therefore having an annulus sealing element helps ensure a tight seal. However, the chamber may have a different profile such that the outer shape of the sealing element's cross section is shaped to match the cross section of the chamber. For instance, the shape may be rectangular, hexagonal, or any other shape. In other arrangements, the inner shape of the cross section of the sealing element (i.e. the shape of the void) may be rectangular, hexagonal, or any other shape if the shape of the consumable cartridge is not cylindrical.

In some arrangements, the valve may be a check valve. In this way, by having a non-return valve ingress of aerosol may be prevented into the internal components of the device. The valve may preferably be a low pressure valve. In this way, only low pressure, such as the pressure generated by a user puffing on the device, may be required to active the valve and allow air to flow from the first to second end.

In some arrangements, the valve may be a ball check valve. The ball check valve comprising a ball that blocks the flow in one direction, whilst allowing flow in the other direction. The ball check valve may comprise a seat on which the ball sits when at rest, and a spring. The spring biases the ball such that a seal is formed around the seat. Upon air flow towards the second end of the chamber the pressure may force the ball against the spring moving the ball from the seat thereby allowing airflow only in this direction through the valve. Upon air flow towards the first end of the chamber the ball remains housed in its seat ensuring the valve remains closed, thereby preventing flow in this direction.

Alternatively, the valve may be a diaphragm check valve. The diaphragm check valve may comprise a disc or diaphragm that acts to close the valve such that it sits on a seat. Upon air flow towards the second end of the chamber the diaphragm/disc is not housed on its seat thereby allowing airflow only in this direction through the valve. Upon air flow towards the first end of the chamber the diaphragm/disc is compressed such that it forms a seal around the seat thus closing the valve, thereby preventing flow in this direction.

Preferably, the sealing element comprises a plurality of valves. The plurality of valves may be arranged and positioned around the sealing element. For instance, the plurality of valves may be positioned around a circumference of the sealing element. By having a plurality of valves this enables a high flow rate of air into the device. The circumference may be of the cross section of the sealing element. For instance, when having an annular sealing element the plurality of valves may be arranged around the circumference of the annulus. This enables uniform airflow through the valves around the chamber towards the second end of the chamber. For instance, the sealing element may comprise four valves one at each quadrant of the cross section of the sealing element. In other arrangements, the sealing element may comprise, one, two, three, five, six, eight or more valves.

Preferably the aerosol generation device further comprises a heater, wherein the sealing element is located between the first end of the chamber and the heater. The heater is configured to heat the aerosol generating material to form the aerosol. By having the sealing element located between the first end of the chamber and the heater it can prevent leakage of aerosol from the heater to prevent damage to the components of the device.

The heater may be located towards, or at, the second end of the chamber. The heater may form a heating area at the second end of the chamber. The second end of the chamber may be proximal to the heater and the first end of the chamber may be distal from the heater. The heater may be located at any position between the first and second end of the chamber. However, having the sealing element located between the heater and the first end of the chamber this prevents the aerosol formed by the heater from escaping to other internal component of the device towards the first end of the chamber.

In some arrangements, the heater may comprise an electrical resistor that can be electrically heated when electricity is supplied through the electrical connector. The electrical resistor can provide heat through Ohmic heating. In other instances, the heater may comprise an inductively energizable susceptor material. The heater may be located around the cartridge. Alternatively, the heater may be positioned within the cartridge to provide heat directly to the aerosol generating material.

Preferably, the aerosol generating device is an electronic cigarette. However, in other arrangements, the device may be a nebulizer device, or any other medical device.

Preferably, the aerosol generating device further comprises the consumable cartridge. Preferably, the consumable cartridge is removable. Alternatively, the consumable cartridge may be fixed part of the device. In this arrangement, the cartridge may be refillable or the device may be single use.

The cartridge may comprise the aerosol-generating material contained within the internal compartment. The aerosol-generating material may comprise tobacco. The aerosol-generating material may be solid or semi-solid. The aerosol-generating material may be preferably aerated tobacco mousse or equivalent foamy tobacco substrate. This may include those described in WO 2020/002607 A1.

For instance, they may be formed by mixing of tobacco with aerosol formers (PG, VG, 1.3 PDO). It may also be formed by mixing the tobacco and aerosol formers with flavours and/or binders. Other tobacco materials for use in a heat-not-burn system may be used. For instance those formed from reconstituted tobacco blend material mixed with aerosol formers. In other arrangements, when the aerosol generating device is for medical use, rather than an e-cigarette, the aerosol generating material may be a medicine.

In a further aspect of the invention there may be provided a sealing element configured to be located in a chamber of an aerosol generating device, the sealing element configured to provide a fluidic seal between a consumable cartridge when the consumable cartridge is received in the aerosol generating device, and the internal side wall of a chamber of the aerosol generating device in which the consumable cartridge is received, the sealing element comprising: a valve, the valve configured such that, flow of fluid is prevented through the valve in a direction towards a first end of the chamber at which the consumable cartridge is received.

### DESCRIPTION OF DRAWINGS:

Figure 1A shows a schematic side-on cross section of a portion of a prior art aerosol generation device showing the airflow when a user inhales;
Figure 1B shows a schematic side-on cross section of a portion of a prior art aerosol generation device showing the airflow, when a user is not inhaling, causing leakage of aerosol into the components of the device;
Figure 2A shows a schematic side-on cross section of a portion of an aerosol generation device according to the present invention showing the airflow when a user inhales, the seal allowing airflow to pass through it in one direction;
Figure 2B shows a schematic side-on cross section of a portion of an aerosol generation device according to the present invention showing the airflow when a user is not inhaling showing the seal preventing leakage of aerosol into the components of the device;
Figure 3 shows a schematic top down cross section of the sealing element of the present invention and a consumable cartridge received with the seal, the sealing element having valves arranged around its perimeter to permit one way flow;
Figure 4A shows a schematic outer side-view of an example valve for use in the sealing element according to the present invention;
Figure 4B shows a schematic cross sectional side view of the valve of Figure 4A;
Figure 5A shows a schematic cross sectional side on view of a further example valve for use in the sealing element according to the present invention showing flow of air through the valve;
Figure 5B shows a schematic cross sectional side on view of a further example valve for use in the sealing element according to the present invention showing flow of air being prevented through the valve in the opposite direction to Figure 5A; and
Figure 6 shows a schematic exploded cross-sectional view of the valve of Figures 5A and 5B.

### DETAILED DESCRIPTION

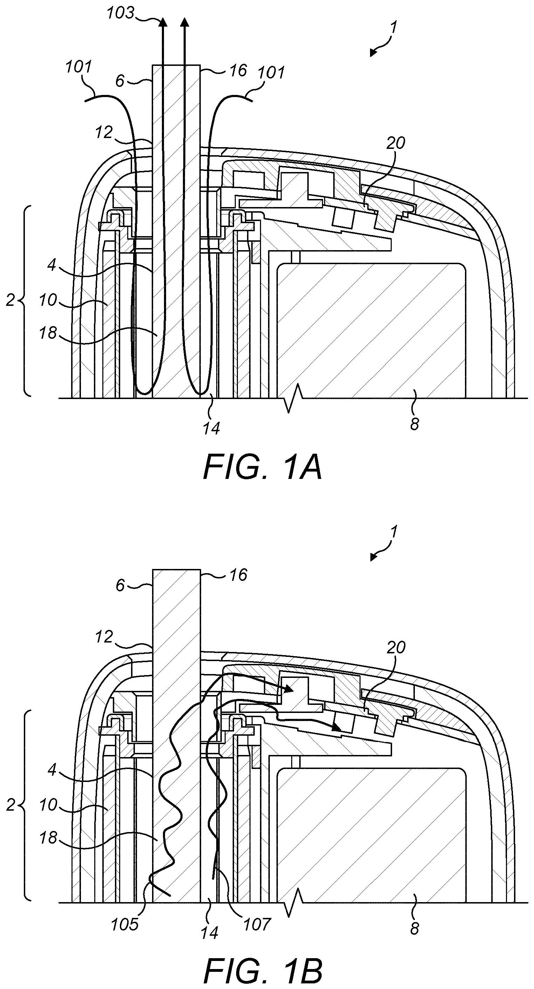

Figures 1A and 1B show schematic side-on cross sectional views of the top portion of a prior art heated tobacco aerosol-generating device 1. A battery 8 is located on one side of the aerosol generating device 1 and a heater 10 located on an opposite side of the aerosol generating device 1. A chamber 4 is present which extends from a first end 12 at the top of the aerosol generating device towards a second end 14. The heater 10 surrounds the chamber 4 at a region towards the second end 14 forming a heating region 2.

A cartridge 6, otherwise known as a consumable or consumable cartridge, is shown located in the chamber 4. The chamber 4 acts as a receiving portion for housing the cartridge 6 when the aerosol generating device 1 is in use. As can be seen, the cartridge 6 has a mouthpiece end 16 which protrudes out of the chamber 4 and a heated end 18 which is in the heating region 2 of the chamber 4. The heated end 18 is perforated allowing airflow between the heating region 2 of the chamber and the cartridge 6.

Electronics 20 are located towards the top of the aerosol generating device 1 above the battery 8. The electronics are responsible for controlling the heater 10 and battery 8 and may provide advanced functionality such as monitoring battery level, puff counter, and other functions.

When in use the cartridge 6 is inserted into the aerosol generating device 1 such that it is located in the chamber 4, as shown in Figures 1A and 1B. The user then places the mouthpiece end 16 of the cartridge 6 into their mouth and inhales. External air, as indicated by reference 101, is drawn into the chamber 4 through first end 12 and into the heating region 2. Electronics 20 control battery 8 to power heater 10 such that heat is supplied to the aerosol generating material located in the cartridge 6 such that it forms an aerosol. The external air enters the cartridge 6 at its heated end 18 forcing the aerosol that has been generated along the cartridge 6 and into the user's mouth, as can be seen by reference 103.

Owing to the fact that the aerosol generating device 1 will not stop heating immediately after the user has finished inhaling, excess aerosol will be formed that is not forced out of the cartridge 6 by the inhale. This aerosol is free to leak in the prior art device shown in Figure 1A and 1B out from the heating region 2 and accumulate around electronics 20, as can be seen by reference 105 and 107. This leakage and ingress of aerosol from the heating region 2 of the device 1 can occur between puffs and shortly after the device has been used. Over time this can cause a build-up of residue that can cause damage to the electronics 20 and other mechanical components of the device thereby leading to malfunctions of these components. The present invention is aimed at avoiding these issues.

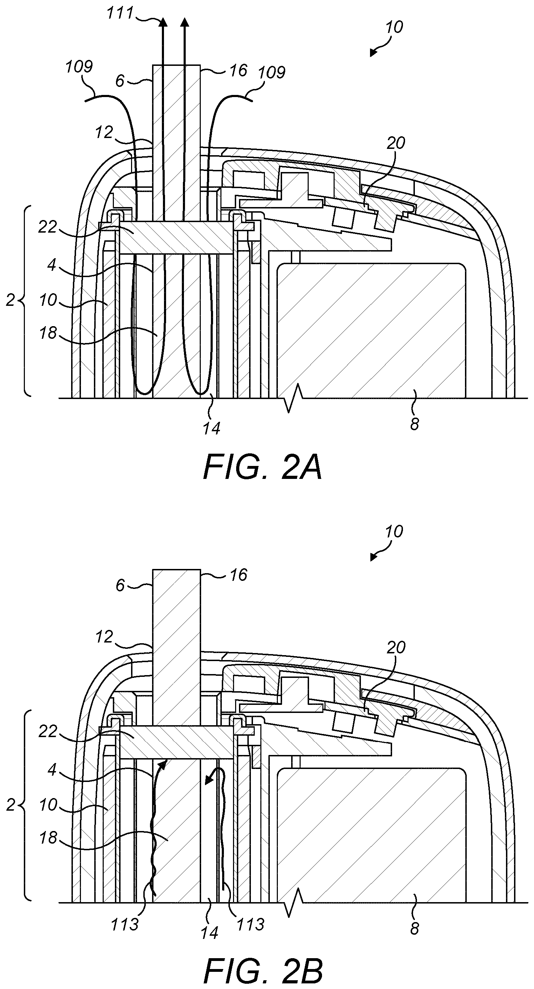

Figures 2A and 2B show schematic side-on cross sectional views of the top portion of a heated tobacco type aerosol-generating device 10 according to the present invention. The aerosol-generating device 10 of the present invention as shown in Figures 2A and 2B is identical to the aerosol generating device 1 of Figures 1A and 1B, with the exception of having sealing element 22. The same components as described above for Figures 1A and 1B have the same reference numerals in Figure 2A and 2B, and therefore will not be discussed in detail again here.

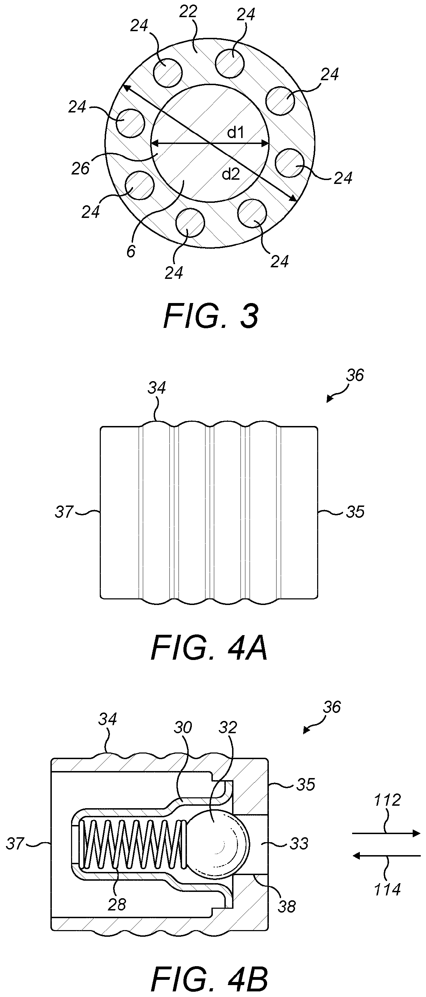

Sealing element 22, otherwise referred to as sealing ring 22, is located within chamber 4 just above the heating region 22 towards the first end 12 of the chamber, but below the electronics 30 (i.e. further from the first end 12 than the electronics 20). Figure 3 shows a schematic top down cross section of the sealing element 22. As can be seen, sealing element 22 has an annular shape. The consumable cartridge 6 is located within the inner portion 26 formed by the sealing element, i.e. in the void or cut-out that is created.

The sealing element 22 has located, equally positioned around its perimeter (i.e. circumference), one-way valves 24. As can be seen in Figure 3 there are eight valves 24. However, there may be fewer, or more valves 24 in other arrangements. The valves are arranged to manage the flow between the first end 12 and second end 14 of the chamber 4. The valves 24 are one way valves, as will be described in more detail in relation to Figures 4 to 6 below.

The annular shape of the sealing element 22 is matched to the circular cross sectional shape of the chamber 4. The sealing element abuts the side wall of the chamber 4 thereby providing an airtight seal. The inner portion 26 of the sealing element is sized and shaped to the circular cross section of the cartridge 6 such that the sealing element abuts the consumable cartridge 6 thereby providing an airtight seal. As can be seen from Figure 3, the inner diameter of the annular sealing element d1 is equal to the diameter of the cartridge 6. The outer diameter of the annular sealing element d2 is equal to the diameter (i.e. width) of the chamber 4.

In this way, the only route for air through the chamber 4 is through the valves 24 of the sealing element 22.

The sealing element 22 is preferably made from Polyether ether ketone (PEEK). Advantageously, this enables the sealing element 22 to withstand chemical and thermal stresses, such as high temperatures. Alternately, or in addition, the sealing element 22 may be comprised of silicone. In other arrangements, the sealing element 22 may be formed of any type of plastic material, ceramic, or rubber.

The cartridge 16 contains an aerosol forming substance, otherwise known as aerosol forming substrate, which preferably contains tobacco. In addition, it may comprise propylene glycol or glycerin which are configured to release an aerosol when heated. The aerosol-generating substrate may be an aerated tobacco mousse or equivalent foamy tobacco substrate. Alternatively, any solid or semi-solid aerosol-generating substrate may be housed within the cartridge 6.

The outer body of the cartridge 6 is made from glass, ceramic, or polymeric material. The cartridge 6 has an end cap at both ends that are air permeable such that they permit the flow of air to enter and leave the cartridge 6. However, in other arrangements the outer body of the cartridge 6 may be air permeable towards either or both ends allowing air flow into and out of the cartridge 6 at the ends of the cartridge 6.

The function of the aerosol generating device 10 of the present invention will now be described. When in use the cartridge 6 is inserted into the aerosol generating device 10 such that it is located in the chamber 4, as shown in Figures 2A and 2B. The cartridge 6 is positioned through the sealing element 22 such that an airtight seal is present between the cartridge 6 and the sealing element 22 and the chamber 4 and the sealing element 22. The user then places the mouthpiece end 16 of the cartridge 6 into their mouth and inhales. External air, as indicated by reference 109, is drawn into the chamber 4 through first end 12 and through the valves 24 of the sealing element 22 such that it enters into the heating region 2. Electronics 20 control battery 8 to power heater 10 such that heat is supplied to the aerosol generating material located in the cartridge 6 such that it forms an aerosol. The external air enters the cartridge 6, which is air permeable at the heated end 18, forcing the aerosol that has been generated along the cartridge 6 and into the user's mouth through the mouthpiece end 16, as can be seen by reference 103.

As outlined above, between puffs, and shortly after use, aerosol that is formed is not flushed out of the device 10, and in prior art devices 1 as explained above in relation to Figure 1B can cause ingress and damage of the internal components of the aerosol generating device 1. In the present invention, as can be seen in Figure 2B, the sealing element 22 prevents flow of this excess aerosol from travelling back up the chamber 4. This is represented by reference 113 in Figure 2B. The airtight seal provided by the sealing element both with the cartridge 6 and the internal walls of the chamber 4 prevents any backflow. Furthermore, as the valves 24 are one-way valves they do not permit flow towards the first end 12 of the chamber. This prevents any ingress of aerosol into and onto the electronic components 20 and other portions of the device above the heating area 2.

The valves 24 are typically microvalves, i.e. they are of such a size that they are small enough to be housed on the sealing element 22. Preferably, the valves are check valves allowing flow in only a single direction, i.e. towards the heating region 2 and not towards the mouthpiece end 12.

Figures 4A and 4B show an example of valve 36 for use in the sealing element 22 of the aerosol generating device 10 of the present invention. Figure 4A shows a schematic outer side-view of a the valve, the valve being a ball check valve 36, with Figure 4B showing a schematic cross sectional side view of the valve of Figure 4A.

Ball check valve 36 has an outer housing 34 having a first end 35 and a second end 37. The outer housing 34 is in contact with the sealing element 22 in which the valve 36 is located forming an airtight seal between the two. Ball check valve 36 has a through-hole 33 that is located in the middle of the first end 35 of the valve with the surrounding portion forming a seat 38 on which a ball 32 sits. The ball 32 is biased into sitting in the seat by spring 28. Spring 28 is held in place by attachment member 30 which attaches back onto the seat 38.

Upon no external flow the ball check valve 36 is in the closed position as shown in Figure 4B, i.e. where ball 32 is sitting on seat 38 and blocking hole 33. Upon external fluid flow travelling along a direction as indicated by arrow 114, (i.e. from the first end 35 to the second end 37 of the valve 36) if the flow is sufficient to overcome the bias of the spring the flow causes the ball 32 to be displaced from the seat 38 thereby allowing fluid to flow through the opening 33 and through the ball check valve 36.

Upon external fluid flow travelling along a direction as indicated by arrow 112, (i.e. from the second end 37 to the first end 35 of the valve 36) the seat prevents the ball 32 from moving such that the opening 34 remains blocked by the ball 32.

Thus, no flow is possible in this direction. By having a spring 28 the valve is in a closed state upon no external flow. However, in other arrangements there may be no spring 28 and instead a flow in direction 112 may cause the valve 36 to close.

The ball check valve 36 is positioned such that the first end 12 of the chamber is closest to the first end 35, and the second end 14 of the chamber is closest to the second end 37. In this way, flow from the second end 14 of the chamber to the first end 12 is prevented through the ball check valve 36, and flow from the first end 12 of the chamber to the second end 14 is permitted so long as the flow is strong enough to overcome the bias of spring 28.

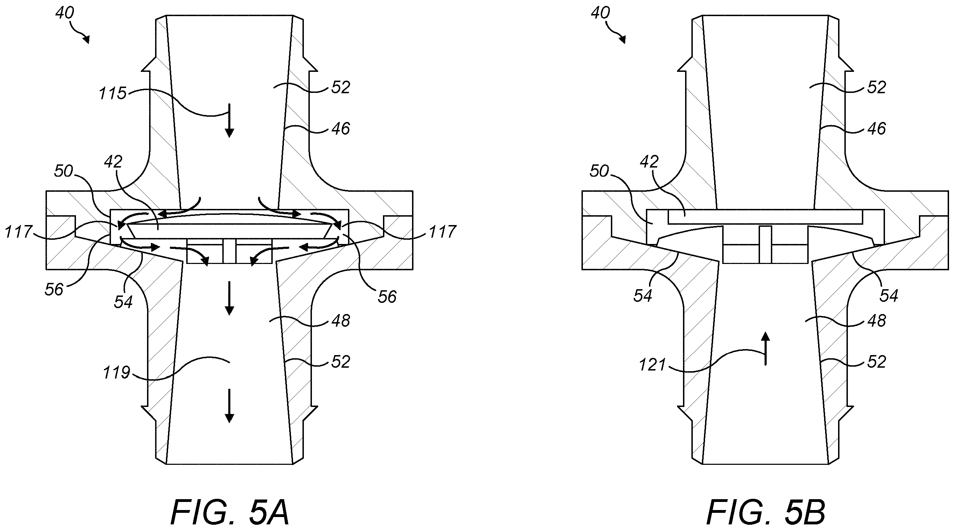

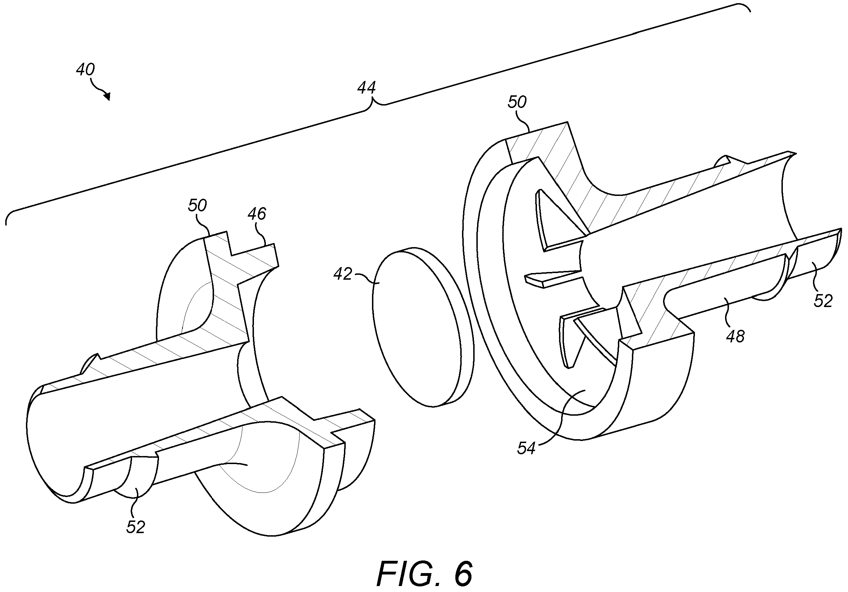

Figure 5A, 5B and 6 show a further example valve 40 for use in the sealing element 22 shown in Figures 2A, 2B and 3.

Figure 5A shows a schematic cross sectional side on view showing flow of air through the valve 40 when the valve is open, and Figure 5B shows a schematic cross sectional side on view showing flow of air prevented through the valve 40 when the valve is closed. Figure 6 shows a schematic exploded cross-sectional view of the valve of Figures 5A and 5B.

The valve 40 shown in Figures 5A, 5B and 6 is a diaphragm check valve 40. It consists of an inlet port 46 and an outlet port 48 and diaphragm 42 between the two. The inlet and outlet ports each have a tube 52 that extends out into a junction 50 where they meet. The junction 50 formed by both the inlet and outlet ports is wider than the tubes 50 such that it can accommodate diaphragm 42. Outlet port 48 also contains a seat 54 in the junction 50 on which the diaphragm 42 sits. The junction 50 has a size such that there is space for the diaphragm to move off from seat 54. The diaphragm 42 is made of an elastomer material, such as rubber or flexible plastic this allows the shape of the diaphragm to slightly change under external pressure to aid in allowing or blocking flow.

Upon no external flow the diaphragm is in an open position and is seated on seat 54. Upon external flow in a direction shown by arrow 115 from inlet 46 to outlet 48 the diaphragm remains in an open position and flow is permitted through channels 56 in the junction 50 such that flow 117 continues into flow 119.

Upon external flow in in a direction shown by arrow 121 from outlet 48 to inlet 46 the diaphragm 42 flexes under the pressure of the flow such that it is displaced from seat 54 and is pushed against the region of the junction 50 connected to the input port 46. This blocks the channels 56 preventing flow through the valve. Once the flow has stopped the diaphragm 42 goes back to its open position.

The diaphragm check valve 40 is located in sealing element 22, as shown in Figures 2A 2B and 3, with the input port 46 located towards the first end 12 of the chamber and the output port 48 located towards the second end 14 of the chamber. This allows flow through the diaphragm check valve 40 from the first end 12 to the second end 14 but prevents flow in the opposite direction.

Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

In the device 10 shown in the Figures the sealing element has an annular cross section. In other arrangements, the sealing element may have a cross section that is a different shape to an annulus. Consumable cartridges are typically cylindrical therefore, having an annulus sealing element helps ensure a tight seal. However, the chamber may have a different profile such that the outer shape of the sealing element's cross section is shaped to match the cross section of the chamber. For instance, the shape may be rectangular, hexagonal, or any other shape. In other arrangements, the inner shape of the cross section of the sealing element (i.e. the shape of the void) may be rectangular, hexagonal, or any other shape if the shape of the consumable cartridge is not cylindrical.

In the Figures described above, the aerosol generating device is an electronic cigarette. However, in other arrangements, the device may be a nebulizer device or any other aerosol generating medical device.

In the above arrangements, eight valves are shown. However, in other embodiments fewer or more valves may be present. For instance, the sealing element 22 may have a single valve. In other arrangements, there may be two, three, four, five, six, or more valves. In addition, the valves shown above are just two example types of valves. Any other type of valve may be used that allows fluid to pass in one direction but not in the opposite direction.

In the above arrangements, the cartridge is described as being removable from the chamber. This allows the cartridge to be replaced, or refilled, when the aerosol generating material is spent. Alternatively, the cartridge may be an integral part of the aerosol generating device. In this way, the aerosol generating device may be single use, or there may be a means for refilling the aerosol generating material once it is spent.

Although a single sealing element 22 is shown in the devices shown in the figures, in other arrangements there may be multiple sealing elements present. Having a plurality of sealing elements may provide an improved seal, such that any fluid that leaks through the first sealing element is stopped by the second sealing element. By having multiple sealing elements they can be positioned dependent on where the elements they are to protect are positioned.

## Claims

1. An aerosol generation device (10), comprising:
a chamber (4) for receiving a consumable cartridge (6), the consumable cartridge comprising an aerosol generating material, the chamber comprising:
a first end through which the consumable cartridge is received;
a second end; and
an internal side wall extending from the first end to the second end, the side wall defining the chamber between the first and second ends;
a sealing element (22) located in the chamber, the sealing element configured to provide a fluidic seal between the consumable cartridge, when received in the chamber, and the internal side wall of the chamber, **characterized in that** the sealing element comprises:
a valve (24), the valve configured such that, flow of fluid is prevented through the valve in a direction towards the first end of the chamber.

2. The aerosol generation device (10) of claim 1, wherein the sealing element is comprised of a plastic material.

3. The aerosol generation device (10) of claim 2, wherein the sealing element (22) is comprised of Polyether ether ketone (PEEK).

4. The aerosol generation device (10) of claim 2 or 3, wherein the sealing element (22) is comprised of silicone.

5. The aerosol generating device (10) of any preceding claim, wherein a first axis is defined between the first and second ends of the chamber (4), and wherein the chamber has a width perpendicular to the first axis, wherein the sealing element (22) has a cross section in a plane perpendicular to the first axis, the cross section having an outline that is substantially matched to the width of the chamber thereby to provide a seal between the sealing element and the internal side wall of the chamber.

6. The aerosol generation device (10) of claim 5, wherein the sealing element (22) has an annular cross section in a plane perpendicular to the first axis, the annular cross section comprising an inner diameter that is configured to receive the consumable cartridge (6), and comprising an outer diameter that is substantially matched to the width of the chamber (4) thereby to provide a seal between the sealing element and the internal side wall of the chamber.

7. The aerosol generation device (10) of any preceding claim, wherein the valve (24) is a check valve.

8. The aerosol generation device (10) of claim 7, wherein the valve (24) is a ball check valve.

9. The aerosol generation device (10) of claim 7, wherein the valve (24) is a diaphragm check valve.

10. The aerosol generation device (10) of any preceding claim, wherein the sealing element (22) comprises a plurality of valves (24).

11. The aerosol generation device (10) of claim 9, wherein the plurality of valves (24) are positioned around a circumference of the sealing element (22).

12. The aerosol generation device (10) of any preceding claim, further comprising a heater, wherein the sealing element (22) is located between the first end of the chamber (4) and the heater.

13. The aerosol generation device (10) of any preceding claim, wherein the aerosol generating device is an electronic cigarette.

14. The aerosol generation device (10) of any preceding claim, further comprising the consumable cartridge (6).

## Patentansprüche

1. Aerosolerzeugungsvorrichtung (10), umfassend:
Eine Kammer (4) zur Aufnahme einer Verbrauchspatrone (6), wobei die Verbrauchspatrone ein Aerosol erzeugendes Material umfasst, wobei die Kammer umfasst:
Ein erstes Ende, durch welches die Verbrauchspatrone aufgenommen wird;
ein zweites Ende; und
eine innere Seitenwand, die sich aus dem ersten Ende zum zweiten Ende erstreckt, wobei die Seitenwand die Kammer zwischen den ersten und zweiten Enden definiert;
ein in der Kammer befindliches Dichtelement (22), wobei das Dichtelement dazu konfiguriert ist, eine fluidische Dichtung zwischen der Verbrauchspatrone, wenn in der Kammer aufgenommen, und der inneren Seitenwand der Kammer bereitzustellen, **dadurch gekennzeichnet, dass** das Dichtelement umfasst:
Ein Ventil (24), wobei das Ventil so konfiguriert ist, dass es Fluidstrom durch das Ventil in eine Richtung gegen das erste Ende der Kammer verhindert.

2. Aerosolerzeugungsvorrichtung (10) nach Anspruch 1, wobei das Dichtelement aus Kunststoffmaterial besteht.

3. Aerosolerzeugungsvorrichtung (10) nach Anspruch 2, wobei das Dichtelement (22) aus Polyetheretherketon (PEEK) besteht.

4. Aerosolerzeugungsvorrichtung (10) nach Anspruch 2 oder 3, wobei das Dichtelement (22) aus Silikon besteht.

5. Aerosolerzeugungsvorrichtung (10) nach irgendeinem vorhergehenden Anspruch, wobei eine erste Achse zwischen den ersten und zweiten Enden der Kammer (4) definiert ist, und wobei die Kammer eine Breite senkrecht zur ersten Achse aufweist, wobei das Dichtelement (22) einen Querschnitt in einer Ebene senkrecht zur ersten Achse aufweist, wobei der Querschnitt einen Umriss aufweist, der im Wesentlichen an die Breite der Kammer angepasst ist, um dadurch eine Abdichtung zwischen dem Dichtelement und der inneren Seitenwand der Kammer bereitzustellen.

6. Aerosolerzeugungsvorrichtung (10) nach Anspruch 5, wobei das Dichtelement (22) einen ringförmigen Querschnitt in einer Ebene senkrecht zur ersten Achse aufweist, wobei der ringförmige Querschnitt einen Innendurchmesser umfasst, der konfiguriert ist, die Verbrauchspatrone (6) aufzunehmen, und einen Außendurchmesser umfasst, der im Wesentlichen der Breite der Kammer (4) angepasst ist, um dadurch eine Abdichtung zwischen dem Dichtelement und der inneren Seitenwand der Kammer bereitzustellen.

7. Aerosolerzeugungsvorrichtung (10) nach irgendeinem vorhergehenden Anspruch, wobei das Ventil (24) ein Rückschlagventil ist.

8. Aerosolerzeugungsvorrichtung (10) nach Anspruch 7, wobei das Ventil (24) ein Kugelrückschlagventil ist.

9. Aerosolerzeugungsvorrichtung (10) nach Anspruch 7, wobei das Ventil (24) ein Membran-Rückschlagventil ist.

10. Aerosolerzeugungsvorrichtung (10) nach irgendeinem vorhergehenden Anspruch, wobei Dichtelement (22) eine Vielzahl von Ventilen (24) umfasst.

11. Aerosolerzeugungsvorrichtung (10) nach irgendeinem vorhergehenden Anspruch 9, wobei die Vielzahl von Ventilen (24) um einen Umfang des Dichtelements (22) herum positioniert sind.

12. Aerosolerzeugungsvorrichtung (10) nach irgendeinem vorhergehenden Anspruch, die weiter ein Heizgerät umfasst, wobei sich das Dichtelement (22) zwischen dem ersten Ende der Kammer (4) und dem Heizgerät befindet.

13. Aerosolerzeugungsvorrichtung (10) nach irgendeinem vorhergehenden Anspruch, wobei die Aerosolerzeugungsvorrichtung eine elektronische Zigarette ist.

14. Aerosolerzeugungsvorrichtung (10) nach irgendeinem vorhergehenden Anspruch, die weiter die Verbrauchspatrone (6) umfasst.

## Revendications

1. Dispositif de génération d'aérosol (10) comprenant :
une chambre (4) pour recevoir une cartouche consommable (6), la cartouche consommable comprenant un matériau de génération d'aérosol, la chambre comprenant :
un premier bout à travers lequel la cartouche consommable est reçue ;
un deuxième bout ; et
une paroi latérale interne s'étendant du premier bout au deuxième bout, la paroi latérale définissant la chambre entre les premier et deuxième bouts ;
un élément d'étanchéité (22) situé dans la chambre, l'élément d'étanchéité étant configuré pour constituer un joint fluidique entre la cartouche consommable, lorsqu'elle est reçue dans la chambre, et la paroi latérale interne de la chambre,
**caractérisé en ce que** l'élément d'étanchéité comprend :
un clapet (24), le clapet étant configuré de façon à empêcher tout écoulement de fluide à travers le clapet dans la direction du premier bout de la chambre.

2. Dispositif de génération d'aérosol (10) selon la revendication 1, l'élément d'étanchéité étant composé d'une matière plastique.

3. Dispositif de génération d'aérosol (10) selon la revendication 2, l'élément d'étanchéité (22) étant composé d'un polyétheréthercétone (PEEK).

4. Dispositif de génération d'aérosol (10) selon la revendication 2 ou 3, l'élément d'étanchéité (22) étant composé de silicone.

5. Dispositif de génération d'aérosol (10) selon une quelconque des revendications précédentes, un premier axe étant défini entre les premier et deuxième bouts de la chambre (4), et la largeur de la chambre étant perpendiculaire au premier axe, l'élément d'étanchéité (22) présentant une section transversale dans un plan perpendiculaire au premier axe, la section transversale présentant un contour correspondant substantiellement à la largeur de la chambre, en formant ainsi un joint entre l'élément d'étanchéité et la paroi latérale interne de la chambre.

6. Dispositif de génération d'aérosol (10) selon la revendication 5, l'élément d'étanchéité (22) présentant une section transversale annulaire dans un plan perpendiculaire au premier axe, la section transversale annulaire comprenant un diamètre intérieur configuré pour recevoir la cartouche consommable (6), et comprenant un diamètre extérieur correspondant substantiellement à la largeur de la chambre (4), en constituant ainsi un joint entre l'élément d'étanchéité et la paroi latérale interne de la chambre.

7. Dispositif de génération d'aérosol (10) selon une quelconque des revendications précédentes, le clapet (24) étant un clapet de retenue.

8. Dispositif de génération d'aérosol (10) selon la revendication 7, le clapet (24) étant un clapet de retenue à bille.

9. Dispositif de génération d'aérosol (10) selon la revendication 7, le clapet (24) étant un clapet de retenue à diaphragme.

10. Dispositif de génération d'aérosol (10) selon une quelconque des revendications précédentes, l'élément d'étanchéité (22) comprenant une pluralité de clapets (24).

11. Dispositif de génération d'aérosol (10) selon la revendication 9, la pluralité de clapets (24) étant positionnée autour d'une circonférence de l'élément d'étanchéité (22).

12. Dispositif de génération d'aérosol (10) selon une quelconque des revendications précédentes, comprenant en outre un appareil de chauffage, l'élément d'étanchéité (22) étant situé entre le premier bout de la chambre (4) et l'appareil de chauffage.

13. Dispositif de génération d'aérosol (10) selon une quelconque des revendications précédentes, le dispositif de génération d'aérosol étant une cigarette électronique.

14. Dispositif de génération d'aérosol (10) selon une quelconque des revendications précédentes, comprenant en outre la cartouche consommable (6).
